# EUROPEAN PATENT APPLICATION

(11) **EP 1 415 595 A1**
(43) Date of publication of application: **06.05.2004**
(21) Application number: 03256710.9
(22) Date of filing: 23.10.2003
(51) Int. Cl.: A61B 6/03, H01J 35/14, H01J 35/30

(54) **Method and apparatus for correcting spherical aberration of an electron beam**

(30) Priority: 23.10.2002 US 65489
(71) Applicant: GE Medical Systems Global Technology Company LLC, Waukesha, Wisconsin 53188-1696 (US)
(72) Inventor: Rand, Roy Edward, Palo Alto California 94306 (US)
(74) Representative: Pedder, James Cuthbert

(57) **Abstract**

Certain embodiments include a computed tomography system including an electron beam source (120) for generating an electron beam (105), an ion clearing electrode (142) for removing ions from the electron beam (105) using electrical fields, an ion trap (144) for allowing ions to accumulate in a downstream region of the electron beam (105) so that the ions do not drift upstream, a beam tube (110) for housing the ion trap (144), and a grounded tube (146) conforming an effective electrical radius of the beam tube (110) to the physical radius of the grounded tube (146) to reduce spherical aberrations in the electron beam (105). Certain embodiments include a method for correcting spherical aberration in an electron beam (105) including producing an electron beam (105, 400), removing ions from the electron beam (105) using electrical fields (400), and allowing ions to accumulate in a downstream portion of the electron beam (105) using an ion trap (144) and a grounded tube (146, 400). The grounded tube (146) adjusts a range of spherical aberration correction of the ion trap (144, 400).

## Description

The present invention generally relates to spherical aberration correction in a diagnostic imaging system. In particular, the present invention relates to correcting spherical aberration of an electron beam in an EBT scanner with an extended range of correction.

Diagnostic imaging systems encompass a variety of imaging modalities, such as x-ray systems, computerized tomography (CT) systems, ultrasound systems, electron beam tomography (EBT) systems, magnetic resonance (MR) systems, and the like. Diagnostic imaging systems generate images of an object, such as a patient, for example, through exposure to an energy source, such as x-rays passing through a patient, for example. The generated images may be used for many purposes. For instance, internal defects in an object may be detected. Additionally, changes in internal structure or alignment may be determined. Fluid flow within an object may also be represented. Furthermore, the image may show the presence or absence of items in an object. The information gained from diagnostic imaging has applications in many fields, including medicine and manufacturing.

In order to help ensure that diagnostic images may be used reliably, image correction is advantageous in diagnostic imaging systems. The image correction in diagnostic imaging systems is important for several reasons, including image quality and system performance. Poor image quality may prevent reliable analysis of the image. For example, a decrease in image contrast quality may yield an unreliable image that is not usable clinically. Additionally, the advent of real-time imaging systems has increased the importance of generating clear, high quality images. The correction of diagnostic images may help to produce a distinct and usable representation of an object.

In CT imaging systems, for example, an object, such as a patient, is x-rayed from a plurality of angles to produce a set of x-ray projections, referred to as a sinogram. Reconstruction in CT imaging calculates a reconstructed two-dimensional image from the sinogram data. The resulting image may be a single slice of the interior of the target object. Multiple slices may also be obtained. Inaccuracies or errors in the CT imaging system may result in blurring, streaking, or introduction of ghost images or artifacts in the resulting image. For example, if an electron beam suffers spherical aberrations, distortion in a scanned image may result.

EBT systems utilize a high energy beam of electrons to strike a target and produce x-rays for irradiating an object to be imaged. The point where the electrons strike the target is called the "beam spot". The electron beam may be "tuned" and/or corrected to minimize error and more accurately produce a beam spot.

In order to achieve as high a spatial resolution as possible, it is desirable to produce an electron beam spot that is as small as theoretically possible and compatible with system safety. To produce a small beam spot, electron beam optics should be free of aberrations. Aberrations in a beam optical system may occur when beam focusing forces, due to external components or to ions within the beam itself, are not proportional to the beam radius. Aberrations may cause the electron beam to become non-uniform downstream. Spherical aberrations are created when a focal strength of the electron beam varies with a radius of the electron beam. Spherical aberrations cause forces within the electron beam to become non-uniform (non-linear) and result in haloes surrounding the beam spot. As a result, x-rays produced at the beam spot will also have a halo. A halo surrounding the x-rays will decrease image quality and definition in a resulting image.

Even though spherical aberration may be corrected, correction may not be perfect. Any beam optical devices that produce some aberration should therefore produce as little spherical aberration as possible. Thus, there is a need for an aberration compensation device to allow use of an electron source with superior beam optics.

As described in U.S. Patent Nos. 5,719,914 and 6,208,711, an electron beam is produced by an electron source at the upstream end of a vacuum housing chamber. A large negative potential (e.g., -130 kV) on the cathode of the electron gun accelerates the electron beam downstream along the chamber axis. Further downstream, a beam optical system that includes magnetic focusing, quadrupole, and deflection coils focuses and deflects the beam to scan along an x-ray producing target. The final beam spot at the target is smaller than that produced at the anode of the electron source. The beam spot must be suitably sharp and halo-free in order to minimize degradation of quality in an image obtained by the imaging system.

In the chamber region upstream of the beam optical system, a diverging beam is desired, and the electron beam may advantageously self-expand due to the force created by its own space-charge. By contrast, downstream from the beam optical system, a converging, self-focusing beam is desired to minimize the final beam spot at the x-ray producing target.

As the electron beam passes through the vacuum chamber, the beam ionizes residual or introduced gas therein, producing positive ions. The positive ions are useful in the downstream chamber region where space charge neutralization and a converging beam are desired. In the upstream region, however, the positive ions would be trapped in the negative electron beam unless removed by an external electrostatic field. Without ion-removal, the space-charge for electron beam self-expansion may be neutralized, and the electron beam may destabilize or even collapse.

In order to adjust the electron beam optics, current EBT scanners may incorporate some form of an ion clearing electrode (ICE) system (for example, a single potential ion clearing electrode system (SPICE, U.S. Patent No. 6,208,711), a rotatable ion clearing electrode assembly (RICE), or a periodic ion clearing electrode (PICE)). An ICE system removes positive ions from the electron beam by creating electric fields in the region between the electron source and the beam optical lens system (a magnetic solenoid, for example). Using electric fields to remove positive ions between the electron source and the beam optical lens system advantageously produces an electron beam that is self-repulsive (or self-divergent) in the upstream or first region and that is self-attractive (or self-focusing) in the downstream or second region.

A typical ICE system is terminated by a Positive Ion Electrode (PIE) or ion trap. The PIE or ion trap preferably segregates the first and second regions of the electron beam. The PIE or ion trap prevents ions that are generated by an electron beam from residual gas downstream from the ICE from drifting upstream. The ions are accumulated downstream of the PIE in order to neutralize the beam's space charge.

The PIE is typically a washer-shaped electrode. The PIE is typically coupled to a high positive potential. The magnitude of the PIE potential may be used to determine the relative lengths of the upstream and downstream beam regions. Further, a suitably high PIE potential may prevent ions created downstream from drifting into the upstream region. The PIE may cause a paraboloidal boundary to form between the space-charge-dominated portion of the electron beam in the ICE and the neutralized portion of the electron beam downstream. The paraboloidal boundary produces spherical aberration in self-focusing forces of the electron beam. As mentioned in U.S. Patent No. 5,719,914, spherical aberration may be controlled by adjusting a voltage applied to the PIE. PIE voltage may be used to cancel or correct spherical aberration produced by other beam line elements, such as an electron source.

However, correction of spherical aberration by adjusting PIE voltage with reasonable applied potentials has both an upper limit and a lower limit. The upper limit of spherical aberration correction may be extended by increasing the aperture of the PIE. In principle, the lower limit may be extended by increasing the voltage applied to the PIE, but even a small extension of the lower limit may require an order of magnitude higher voltage. An alternative approach of decreasing the lower limit by using a smaller PIE aperture is limited by a need to maintain a safe separation of the electron beam and the electrode. Thus, if an electron source were designed with superior beam optics and less spherical aberration, current PIE designs would be unable to compensate for the spherical aberration. In fact, current PIE designs may worsen spherical aberration in an electron beam with superior optics due to limitations on the upper and lower limits of spherical aberration correction in current PIEs.

Thus, a need exists for a method and apparatus for extending lower and upper limits of spherical aberration correction to improve image quality with current beam optics and future superior electron beam optics. Additionally, there is a need for a method and system for extending lower and upper spherical aberration limits with reasonable applied voltages to eliminate unreasonable high magnitude potentials necessary with current attempts. There is a further need for a method and system for adjusting spherical aberration correction while maintaining a safe separation between the electron beam and the electrode. Therefore, a need exists for a method and apparatus for correcting spherical aberration of an electron beam in an EBT scanner with an extended range of correction.

Certain embodiments of the invention include a computed tomography system. The system includes an electron beam generator for generating an electron beam, an ion clearing electrode for removing ions from the electron beam using electrical fields, an ion trap for accumulating ions in a downstream region of the electron beam so that the ions do not drift upstream, a beam tube for housing the ion trap and ion clearing electrode, and a grounded tube conforming an effective radius of the beam tube to the physical radius of the grounded tube to reduce spherical aberrations in the electron beam. In certain embodiments, the grounded tube is a non-magnetic grounded tube. The radius of the grounded tube extends a lower limit of spherical aberration correction of the ion trap. In certain embodiments, the ion trap is a positive ion electrode including an aperture through which the electron beam passes. The size of the aperture is adjusted to provide safe clearance from the electron beam and defines an upper limit of spherical aberration correction of the ion trap. The ion trap uses a voltage to create a neutralization boundary in the electron beam to allow ions to accumulate in the downstream region of the electron beam. The grounded tube decreases the voltage to be applied to the ion trap. The system may also include beam optics to aim and/or focus the electron beam. The system may also include a target producing x-ray radiation in response to impact by the electron beam and a detector for detecting the x-ray radiation produced by the target.

Certain embodiments include a method for correcting spherical aberration in an electron beam having an upstream region and a downstream region. The method includes producing an electron beam, removing ions from the electron beam using electrical fields in the upstream region of the electron beam, and allowing ions to accumulate in the downstream portion of the electron beam using an ion trap and a grounded tube. The grounded tube adjusts a range of spherical aberration correction of the ion trap. The method also includes adjusting an aperture in the ion trap to adjust the range of spherical aberration correction of the ion trap. Additionally, a voltage may be applied to the ion trap to form a neutralization boundary to trap the ions downstream in the electron beam. The voltage applied to the ion trap to form the neutralization boundary decreases as a radius of the grounded tube decreases. The ion trap may be a positive ion electrode. In certain embodiments, the electrical fields used to remove ions are generated by an ion clearing electrode.

The invention will now be described in greater detail, by way of example, with reference to the drawings, in which:-
Figure 1 illustrates an EBT imaging system formed in accordance with an embodiment of the present invention.
Figure 2 illustrates a portion of an electron beam housing formed in accordance with an embodiment of the present invention.
Figure 3 illustrates an electrode assembly formed in accordance with an embodiment of the present invention.
Figure 4 depicts a flow diagram for a method for correcting spherical aberration in an electron beam according to an embodiment of the present invention.
Figure 5 illustrates a graph depicting spherical aberrations versus PIE voltage based on grounded tube radius in accordance with an embodiment of the present invention.
Figure 6 depicts spherical aberration of the electron beam versus grounded tube radius based on PIE voltage in accordance with an embodiment of the present invention.
Figure 7 compares a conventional system using PIE to an improved system using PIE with a grounded tube in accordance with an embodiment of the present invention.
Figure 8 illustrates the positioning of the PIE and the grounded tube in the EBT imaging system according to an embodiment of the present invention.

For the purpose of illustration only, the following detailed description references a certain embodiment of an Electron Beam Tomography (EBT) imaging system. It is understood that the present invention may be used with other imaging systems and other electron beam systems.

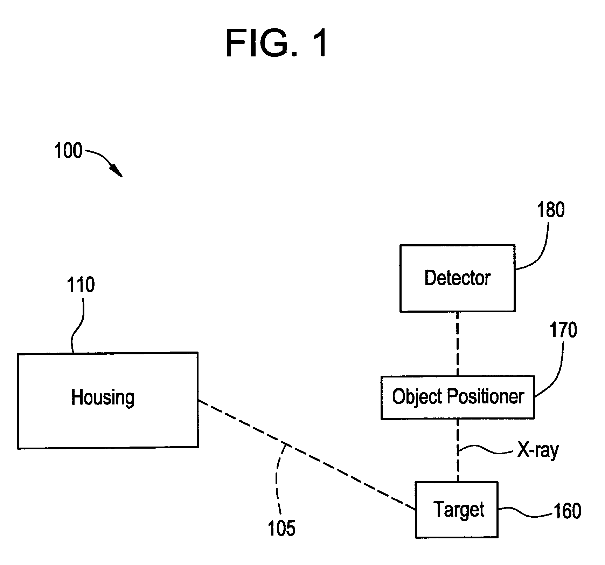

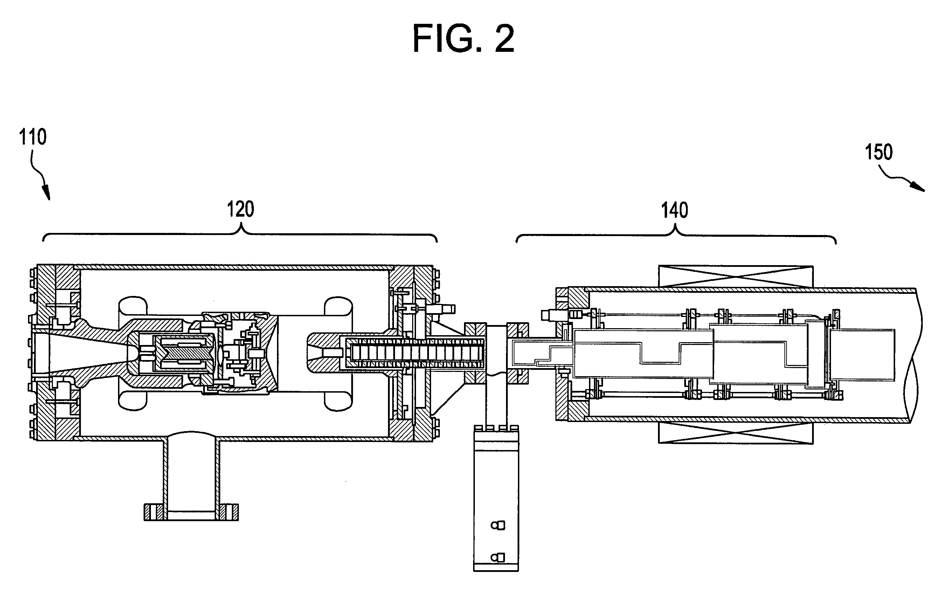

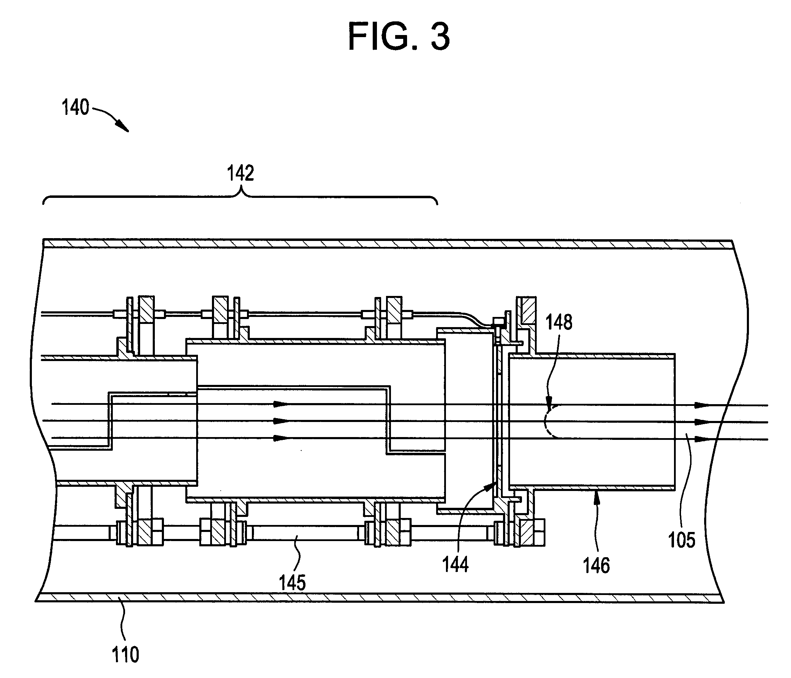

Figure 1 illustrates an EBT imaging system 100 formed in accordance with an embodiment of the present invention. The EBT system 100 includes a housing 110, a target 160, an object positioner 170, and a detector 180. As illustrated in greater detail in Figure 2, the housing 110 includes an electron source 120, an electrode assembly 140, and beam optics 150. As shown in Figure 3, the electrode assembly 140 includes an ion clearing electrode (ICE) 142, a Positive Ion Electrode (PIE) 144, and a grounded tube 146.

The electron source 120 provides a source of electrons that may be used to form an electron beam 105 for use in imaging. The electron beam 105 from the electron source 120 is used to produce radiation, such as x-ray radiation, for use in imaging at the target 160. The electron source 120 may be connected to a voltage generator (not pictured) and is used to generate an electron beam 105. For example, the electron beam 105 is generated at the cathode of the electron source 120 in response to a -130 kV voltage from the voltage generator.

The electrode assembly 140 controls positive ions in an upstream region of the electron beam 105 using the ICE 142, washer-shaped PIE 144, and grounded tube 146. For example, the electrode assembly 140 may control positive ions by surrounding and subjecting an electron beam 105 to electric fields transverse to the electron beam 105 velocity that advantageously extract positive ions from the electron beam 105 while minimizing net electron beam 105 deflection.

The ion clearing electrode (ICE) 142 removes positive ions from the electron beam 105. The ICE 142 may be a multi-sided rotatable field ion clearing electrode (RICE), multiple multi-sided ion clearing electrodes (ICE), a periodic axial field ion controlling electrode (PICE), or a single potential ion clearing electrode (SPICE), for example. The ICE 140 is terminated by the PIE or ion trap 144.

The SPICE, for example, extracts positive ions from the electron beam 105 using an ion clearing electrode assembly that is operable from a single source of operating potential and produces essentially zero net beam deflection and displacement. The SPICE is normally installed inside a magnetic solenoid (focusing) lens, concentric about the Z-axis of the system 100, and uses two basic arrays of electrode cross-sections that alternate along the Z-axis to generate electric fields to cancel or minimize focusing and aberration-producing fields around the electron beam 105.

The PIE 144 or ion trap prevents ions generated by residual gas in the electron beam 105 downstream from the ICE 142 from drifting upstream into the ICE 142. The ions are accumulated in the downstream portion of the electron beam 105 to neutralize space-charge exhibited by the electron beam 105. The PIE 144 may be a washer-shaped electrode. A voltage potential applied to the PIE 144 produces a paraboloidal neutralization boundary 148 to form between the space-charge-dominated portion of the electron beam 105 in the ICE 142 and the neutralized portion of the electron beam 105 downstream. Ions accumulate downstream from the paraboloidal neutralization boundary 148. The accumulated ions neutralize the electron beam 105 and cause the electron beam 105 to be self-focusing.

The paraboloidal boundary 148 between the space-charged beam and the neutralized beam causes beam 105 self-focusing forces which are proportional to beam 105 radius cubed. Focusing forces that are proportional to the beam 105 radius cubed are a classical condition for spherical aberrations. The magnitude of the spherical aberrations is proportional to the axial extent of the neutralization boundary 148. The extent the boundary 148 is controlled by the voltage applied to the PIE 144 and by the dimensions of the PIE 144 and the grounded tube 146. A voltage potential applied to the PIE 144 may be used to cancel or correct spherical aberrations produced by other beam line elements, such as the electron source 120, by modifying the paraboloidal neutralization boundary 148 (i.e., reducing the extent of the boundary 148).

The grounded tube 146 shifts the range of spherical aberration correction of the PIE 144 to lower values using an applied potential. The grounded tube 146 is a metal tube, such as aluminum, non-magnetic stainless steel, or copper, for example. The grounded tube 146 is grounded in the system 100 by attachment to an ICE support frame 145 or the inside wall of the housing or beam tube 110, for example. In an embodiment, the grounded tube 146 is an aluminum tube having a length of 80 mm, a radius of 30 mm, and a thickness of 1.6 mm. The dimensions, composition, and positioning of the grounded tube 146 may vary.

The grounded tube 146 is positioned downstream from the PIE 144 in the beam tube 110. The grounded tube 146 is sufficiently spaced from the PIE 144 to prevent arcing between the grounded tube 146 and the PIE 144 (for example, spacing of a few millimeters). For example, a spacing of two millimeters or more may prevent arcing between the grounded tube 146 and the PIE 144 at an applied voltage potential of 1000 V. In a certain embodiment, the space between the grounded tube 146 and the PIE 144 is smaller than the grounded tube 146 and the PIE 144 radii (for example, less than 6 mm). In a certain embodiment, the end of the grounded tube 146 facing the downstream region of the electron beam 105 is several electron beam 105 diameters beyond the paraboloidal neutralization boundary 148 formed by the PIE 144.

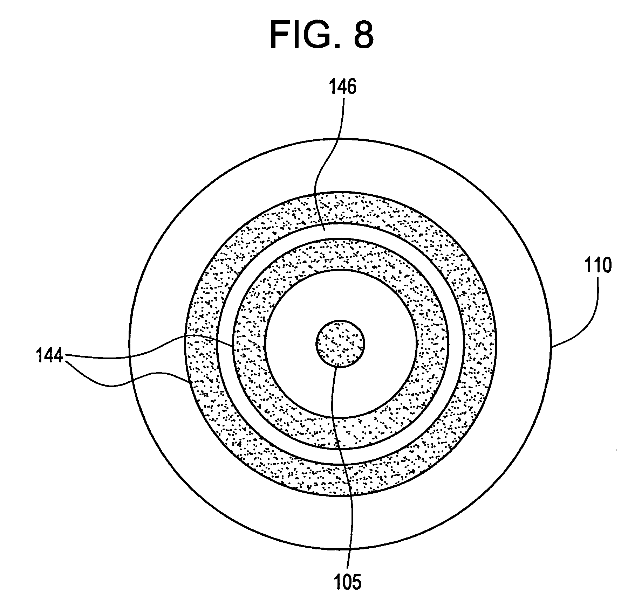

Figure 8 illustrates the positioning of the PIE 144 and the grounded tube 146 in the EBT imaging system 100 according to an embodiment of the present invention. The grounded tube 146 and the PIE 144 are centered symmetrically around the axis along which the electron beam 105 travels through the beam tube 110. The PIE 144 and the grounded tube 146 are mounted in the ICE support frame 145 within the beam tube 110. in an embodiment, the grounded tube 146 and the PIE 144 are each attached to the support frame 145 by three fasteners that are spaced 120 degrees apart.

When the grounded tube 146 is positioned downstream from the PIE 144, the radius of the grounded tube 146 (for example, 30 mm) becomes the effective "electrical" radius of the beam tube or housing 110 surrounding the electron beam 105 downstream from the PIE 144. That is, when the grounded tube 146 is present downstream from the PIE 144, the grounded tube 146 becomes the beam tube 110 from an electrostatic perspective. However, from a vacuum or physical perspective, the beam tube 110 has a larger radius than the grounded tube 146 (for example, 76 mm). If the grounded tube 146 is not present, the electrical radius (radius in an electrostatic sense) and the vacuum/physical radius of the beam tube 110 are the same (for example, 76 mm). The beam tube provides vacuum pumping for the ICE 142/PIE 144 system.

In certain embodiments, the electrostatic potential at the paraboloidal neutralization boundary 148 produced by the PIE 144 is equal to the voltage potential due to the PIE 144 plus the voltage potential of the electron beam 105. Downstream from the PIE 144, the magnitude of the voltage potential of the electron beam 105 decreases as the radius of the grounded tube 146 decreases. If the grounded tube 146 radius were reduced to equal the electron beam 105 radius (i.e., the beam 105 would touch the grounded tube 146), the voltage potential of the edge of the electron beam 105 would be zero.

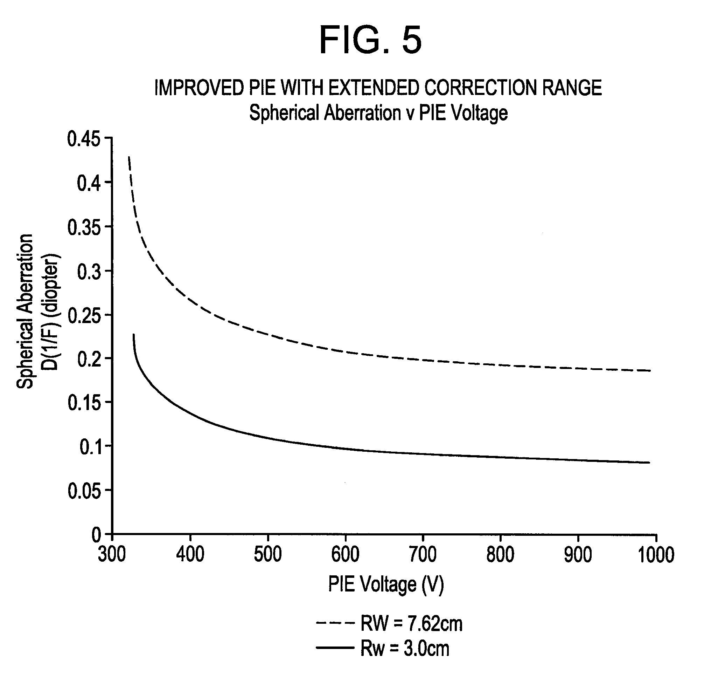

Spherical aberrations of the electron beam 105 are affected by the voltage potential of the PIE 144, the radius of the PIE 144, and the radius of the beam tube 110. Changes to the voltage potential applied to the PIE 144 may be impractical beyond a certain threshold. Modification of the PIE 144 aperture may also be of limited effectiveness after a certain threshold. The introduction of the grounded tube 146 effectively reduces the radius of the portion of the beam tube 110 downstream from the PIE 144 in electrical terms, while leaving the physical beam tube 110 radius unchanged. That is, the effective electrical radius of the beam tube 110 conforms to the physical radius of the grounded tube 146. The grounded tube 146 restricts the potential of the electron beam 105 and helps to minimize spherical aberrations downstream from the PIE 144. As further described below in relation to Figures 5, 6, and 7, for the same spherical aberration (the same neutralization boundary length, for example), the potential due to the PIE 144 and the voltage applied to the PIE 144 decrease as the radius of the grounded tube 146 and the magnitude of the potential due to the beam 105 decrease.

The electron beam 105 is focused by the beam optics 150. The beam optics 150 may adjust radius, angle, and/or timing of the electron beam 105, for example. The beam optics 150 may include a quadrupole coil, a deflection coil, and a magnetic lens. The coils focus and shape the electron beam 105 to impact the target 160 for use in imaging.

The target 160 produces radiation, such as x-ray radiation, upon contact by the electron beam 105. The location at which the electron beam 105 strikes the target 160 is referred to as the beam spot. The target 160 is struck by the beam 105 at the beam spot, and x-rays are produced. The x-rays travel away from the target 160. The target 160 may be a metal target, such as a tungsten target, for example.

The object positioner 170 positions an object to be imaged at least partially in the path of the radiation emitted from the target 160 at the beam spot. The object on the object positioner 170 is irradiated as the x-rays travel from the target 160. The object positioner 170 may be movable or immovable and may position the object horizontally and/or vertically.

The detector 180 detects radiation impinging upon it from the target 160. X-rays from the target 160 irradiate the object on the object positioner 170 and then strike the detector 180. X-rays passing through the object are attenuated to varying degrees depending on the density of the matter through which the x-rays pass. X-rays impacting on the detector 180 generate an electrical response corresponding to the intensity of the attenuated radiation. A diagnostic image is formed from the electrical response.

In operation, the voltage generator and the electron source 120 generate an electron beam 105 from the cathode of the electron source 120. The electron beam 105 passes from the cathode of the electron source 120 to the electrode assembly 140. Within the electrode assembly 140, electrodes in the ICE 142 produce electric fields that remove positive ions from the electron beam 105. Thus, the electron beam 105 is charged within the ICE 142.

Next, the electron beam 105 enters the PIE 144. The PIE 144 traps or confines ions to the downstream portion of the electron beam 105. The PIE 144 produces a boundary 148 that blocks the ions. The ions neutralize the electron beam 105 downstream from the ICE 142.

Spherical aberrations are introduced by ion clearing and ion trapping and by the electron source 120, as well as other components. The PIE 144 voltage controls the spherical aberrations due to ion trapping. The PIE 144 may cancel spherical aberrations in the electron beam 105 due to other components. However, if the degree of spherical aberrations to be cancelled becomes excessive, a large voltage must be applied to the PIE 144 to correct the spherical aberrations. The PIE 144 voltage may become too large. However, with the introduction of the grounded tube 146, the PIE 144 and grounded tube 146 cooperate to adjust the spherical aberration characteristics and provide a wider range for correction of spherical aberrations.

When used alone, the PIE 144 has an inherent upper limit and lower limit to its spherical aberration correction capabilities. The upper limit of correction is extended by increasing the aperture or opening of the PIE 144. The lower correction limit is extended by increasing the voltage applied to the PIE 144.

Without the grounded tube 146, a small extension of the lower limit requires an increase in applied voltage by an order of magnitude. The grounded tube 146 allows extension of the lower limit of spherical aberration correction based on the radius of the grounded tube 146 in relation to the beam tube 110 housing the electrode assembly 140.

After the electron beam 105 has passed through the PIE 144 and the grounded tube 146 and the beam optical system 150, spherical aberration in the electron beam 105 has been minimized. The PIE 144 with the grounded tube 146 reduces the halo surrounding the electron beam 105 at the beam spot caused by spherical aberrations. Thus, the electron beam 105 that strikes the target 160 produces x-rays from the target 160 that possess a reduced halo.

X-rays generated at the target 160 travel outwards through the object positioner 170 and irradiate an object positioned on the object positioner 170. The x-rays that are not blocked by the object then impinge upon the detector 180. Signals are generated based on the strength of the x-rays impacting the detector 180. The signals are used to produce an image. X-rays with reduced halo help to improve image quality in the resulting image.

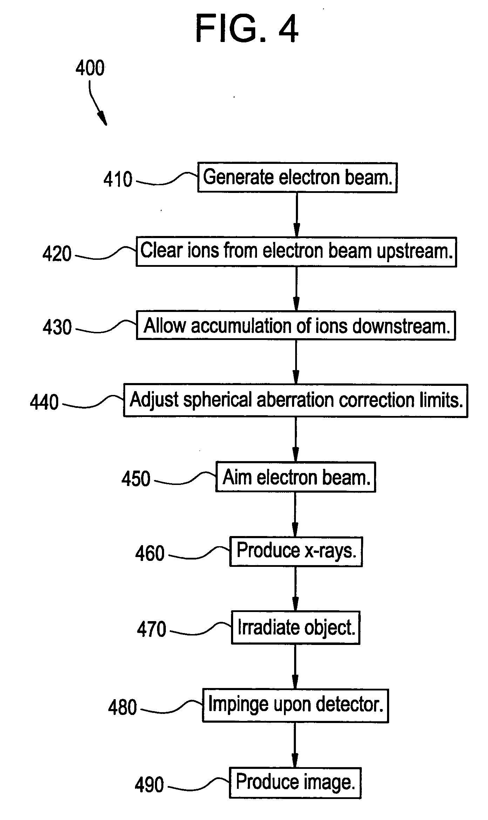

Figure 4 depicts a flow diagram for a method 400 for correcting spherical aberrations in the electron beam 105 according to an embodiment of the present invention. First at step 410, an electron beam 105 is generated. Next, at step 420, ions are cleared from the upstream electron beam 105 through electrical fields that are produced by the ICE 142. At step 430, ions are allowed to accumulate in a downstream portion of the electron beam 105. Ion accumulation beyond the paraboloidal neutralization boundary 148 occurs automatically. Thus, the portion of the electron beam 105 downstream from the neutralization boundary 148 is largely neutralized and contains a number of ions equal to the number of electrons. For example, a washer-shaped ion trap, such as the PIE 144, with an opening in the center, may be used to create a paraboloidal boundary 148 blocking the ions from moving upstream.

However, ion trapping has limits in spherical aberration correction. Therefore, at step 440, the correction limits of the ion trap, such as the PIE 144, are shifted. For example, the upper limit of the PIE 144 may be adjusted by adjusting the size of the opening or aperture in the PIE 144. Additionally, the lower limit of spherical aberration correction of the PIE 144 may be adjusted by placing a grounded tube 146 downstream from the PIE 144. As discussed further below in relation to Figure 5, the dimensions of the grounded tube 146 decrease the limits of spherical aberration correction.

An upper limit of spherical aberration correction is increased by widening an aperture of an ion trap, such as the PIE 144, through which an electron beam 105 passes. The ion trap allows ions to accumulate in the electron beam 105 at a downstream portion of the electron beam 105. A lower limit of spherical aberration correction is expanded by positioning a grounded tube 146 beyond the ion trap to shift the range of correction of the ion trap to lower values. The dimensions of the grounded tube 146 affect electrical interaction between the ion trap and the electron beam 105 and lower the range of spherical aberration correction provided by the ion trap.

Then, at step 450, the corrected electron beam 105 is aimed and/or focused by the beam optics 150. At step 460, the electron beam 105 impacts the target 160 and produces x-rays. Next, at step 470, the x-rays travel from the target 160 and pass through an object located on the object positioner 170, irradiating the object. At step 480, the x-rays impinge upon the detector 180. The x-rays produce signals at the detector 180 in proportion to the intensity with which the x-rays arrive at the detector 180 after irradiating the object on the object positioner 170. Finally, at step 490, the signals are used to produce an image representative of the object through which the x-rays passed.

Figure 5 illustrates a graph depicting spherical aberrations versus PIE 144 voltage based on different grounded tube 146 radii. Figure 5 shows a shift of voltage for constant spherical aberrations as the grounded tube 146 radius decreases. At a smaller physical grounded tube 146 radius, and thus a smaller effective electrical radius of the beam tube 110, a lower voltage potential applied to the PIE 144 achieves the same reduction of spherical aberrations in the electron beam 105 as a higher voltage potential applied with a larger effective electrical radius. In Figure 5, the spherical aberrations at the edge of the electron beam 105 is the same at 0.2 diopter with a PIE 144 voltage of 710 V and a grounded tube 146 radius of 7.62 cm and with a PIE 144 voltage of 330 V and a grounded tube 146 radius of 3.0 cm. Thus, a small grounded tube 146 radius allows a given reduction in spherical aberration at a lower applied voltage potential.

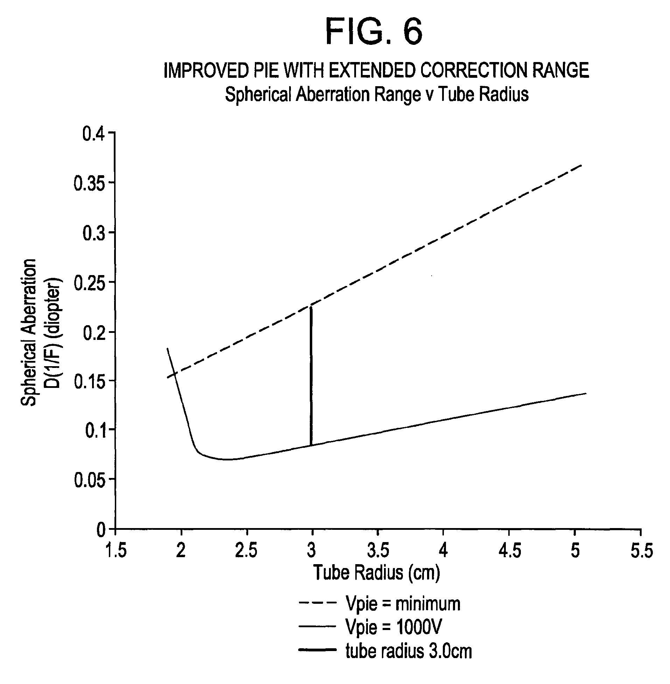

Figure 6 depicts spherical aberration of the electron beam 105 versus grounded tube 146 radius based on PIE 144 voltage. Figure 6 shows how spherical aberration generally increases as the radius of the grounded tube 146 increases for a given voltage potential applied to the PIE 144. At a constant voltage, a decrease in the radius of the grounded tube 146, and thus a decrease in the effective electrical radius of the beam tube 110, allows for an extended range of reduction in spherical aberrations of the electron beam 105. Figure 6 illustrates spherical aberration versus grounded tube 146 radius for a PIE 144 applied potential of 1000V and a minimum applied PIE 144 potential. For example, with a grounded tube 146 radius of 3.0cm, spherical aberration correction of the electron beam 105 is 0.225 diopter at a minimum PIE 144 voltage and 0.08 diopter at a PIE 144 voltage of 1000V.

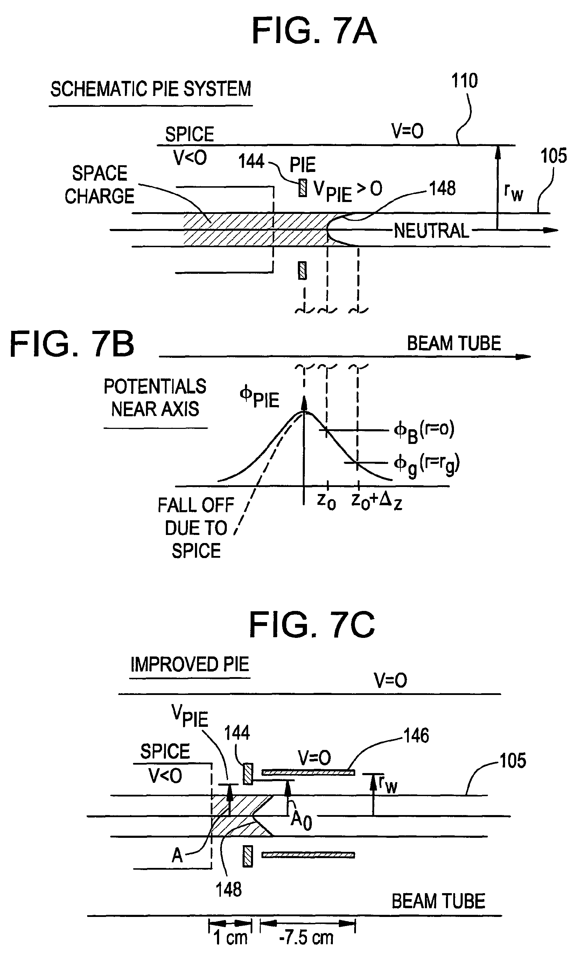

Figure 7 compares a conventional system using PIE 144 to an improved system using PIE 144 with a grounded tube 146 in accordance with an embodiment of the present invention. Figure 7(a) illustrates a conventional system using PIE 144 with a large radius *r*_{*w*} between the center of the electron beam 105 and the wall of the beam tube. Figure 7(b) graphs PIE 144 potential near the z-axis of the PIE 144 and the neutralization boundary 148 formed by voltage applied to the PIE 144. The voltage potential due to the PIE 144 is shown with respect to distance along the z-axis. The PIE 144 voltage potential and the extent of the neutralization boundary 148 along the z-axis of the electron beam 105 impact spherical aberrations within the electron beam 105.

An improved PIE 144 with the grounded tube 146 is shown in Figure 7(c). As shown in Figure 7(c), the radius *r*_{*w*} is greatly reduced from the center of the electron beam 105 to the wall of the grounded tube 146, rather than the wall of the beam tube or housing 110. Additionally, the neutralization boundary 148 is formed by the PIE 144 closer to the PIE 144 than in the prior art and is of reduced extent. By reducing the radius and shortening the extent of the boundary 148 along the z-axis of the electron beam 105, spherical aberrations may be reduced. The addition of the grounded tube 146 improves the reduction and ease of reduction in spherical aberration of the electron beam 105 and, thus, improves resulting image quality as well.

For the sake of good order, various aspects of the invention are set out in the following clauses: -
1. A method for correcting spherical aberration in an electron beam (105) having an upstream region and a downstream region, said method comprising:
   producing an electron beam (105, 400);
   removing ions from the electron beam (105) using electrical fields in the upstream region of the electron beam (105, 400);
   allowing ions to accumulate in the downstream region of the electron beam (105 using an ion trap (144) and a grounded tube (146, 400); and
   adjusting a range of spherical aberration correction of the ion trap (144) with the grounded tube (146, 400).
2. The method of clause 1, further comprising adjusting an aperture in the ion trap (144) to adjust the range of spherical aberration correction of the ion trap (144).
3. The method of clause 1, further comprising applying a voltage to the ion trap (144) to form a neutralization boundary (148) to trap the ions accumulated in the downstream region of the electron beam (105).
4. The method of clause 3, wherein the voltage applied to the ion trap (144) to form the neutralization boundary (148) decreases as a radius of the grounded tube (146) decreases.
5. The method of clause 1, wherein the ion trap (144) comprises a positive ion electrode (144).
6. The method of clause 1, wherein the electrical fields are generated by an ion clearing electrode (142).
7. A computed tomography system, said system comprising:
   an electron beam source (120) for generating an electron beam (105);
   an ion clearing electrode (142) for removing ions from said electron beam (105) using electrical fields;
   an ion trap (144) for allowing ions to accumulate in a downstream region of said electron beam (105) so that said ions do not drift upstream;
   a beam tube (110) for housing said ion trap (144); and
   a grounded tube (146), located downstream of said ion trap (144), said grounded tube (146) being electrically grounded, an effective electrical radius of said beam tube (110) conforming to a physical radius of said grounded tube (146) to reduce spherical aberration in said electron beam (105).
8. The system of clause 7, wherein said grounded tube (146) comprises a non-magnetic grounded tube (146).
9. The system of clause 7, wherein a radius of said grounded tube (146) extends a lower limit of spherical aberration correction of said ion trap (144).
10. The system of clause 7, wherein said ion trap (144) comprises a positive ion electrode (144), said positive ion electrode (144) including an aperture through which said electron beam (105) passes.
11. The system of clause 10, wherein size of said aperture is adjusted to adjust an upper limit of spherical aberration correction of said ion trap (144).
12. The system of clause 7, wherein said ion trap (144) uses a voltage to create a neutralization boundary (148) to trap ions in said downstream region of said electron beam (105).
13. The system of clause 12, wherein said grounded tube (146) decreases said voltage applied to said ion trap (144) for a certain spherical aberration correction.
14. The system of clause 7, further comprising beam optics (150) to at least one of aim and focus said electron beam (105).
15. The system of clause 7, further comprising a target (160) producing x-ray radiation in response to impact by said electron beam (105).
16. The system of clause 15, further comprising a detector (180) for detecting said x-ray radiation produced at said target (160).
17. A method for reducing spherical aberration in a computed tomography scanner, said method comprising:
   increasing an upper limit of spherical aberration correction by widening an aperture of an ion trap (144), the ion trap (144) allowing ions to accumulate in an electron beam (105) at a downstream portion of the electron beam (105) using an applied potential;
   extending a lower limit of spherical aberration correction by positioning a grounded tube (146) beyond the ion trap (144); and
   passing an electron beam (105) through the ion trap (144) and the grounded tube (146).
18. The method of clause 17, wherein the grounded tube (146) decreases the applied potential for spherical aberration correction.
19. A system for correcting spherical aberration in an electron beam (105), said system comprising:
   an ion trap (144) having a voltage for applying a potential to an electron beam (105) to allow ions to accumulate in said electron beam (105) in a portion of said electron beam (105) downstream from said ion trap (144) in order to reduce spherical aberration in said electron beam (105); and
   a grounded tube (146) for extending a range of spherical aberration reduction in relation to at least one of dimension and position of said grounded tube (146), said grounded tube (146) reducing said voltage applied to said ion trap (144).
20. The system of clause 19, wherein said voltage applied to said ion trap (144) decreases as a radius of said grounded tube (146) decreases.
21. The system of clause 19, wherein said range of spherical aberration reduction expands as size of an opening in said ion trap (144) expands.

## Claims

1. A method for correcting spherical aberration in an electron beam (105) having an upstream region and a downstream region, said method comprising:
producing an electron beam (105, 400);
removing ions from the electron beam (105) using electrical fields in the upstream region of the electron beam (105, 400);
allowing ions to accumulate in the downstream region of the electron beam (105 using an ion trap (144) and a grounded tube (146, 400); and
adjusting a range of spherical aberration correction of the ion trap (144) with the grounded tube (146, 400).

2. The method of claim 1, further comprising adjusting an aperture in the ion trap (144) to adjust the range of spherical aberration correction of the ion trap (144).

3. The method of claim 1, further comprising applying a voltage to the ion trap (144) to form a neutralization boundary (148) to trap the ions accumulated in the downstream region of the electron beam (105).

4. The method of claim 3, wherein the voltage applied to the ion trap (144) to form the neutralization boundary (148) decreases as a radius of the grounded tube (146) decreases.

5. The method of claim 1, wherein the ion trap (144) comprises a positive ion electrode (144).

6. The method of claim 1, wherein the electrical fields are generated by an ion clearing electrode (142).

7. A computed tomography system, said system comprising:
an electron beam source (120) for generating an electron beam (105);
an ion clearing electrode (142) for removing ions from said electron beam (105) using electrical fields;
an ion trap (144) for allowing ions to accumulate in a downstream region of said electron beam (105) so that said ions do not drift upstream;
a beam tube (110) for housing said ion trap (144); and
a grounded tube (146), located downstream of said ion trap (144), said grounded tube (146) being electrically grounded, an effective electrical radius of said beam tube (110) conforming to a physical radius of said grounded tube (146) to reduce spherical aberration in said electron beam (105).

8. The system of claim 7, wherein said grounded tube (146) comprises a non-magnetic grounded tube (146).

9. The system of claim 7, wherein a radius of said grounded tube (146) extends a lower limit of spherical aberration correction of said ion trap (144).

10. The system of claim 7, wherein said ion trap (144) comprises a positive ion electrode (144), said positive ion electrode (144) including an aperture through which said electron beam (105) passes.
